# EUROPEAN PATENT SPECIFICATION

(11) **EP 0 571 219 B1**
(45) Date of publication and mention of the grant of the patent: **16.04.1997**
(21) Application number: 93303951.3
(22) Date of filing: 20.05.1993
(51) Int. Cl.: A23L 1/29, A23L 1/308, A23D 9/00, A23D 7/00

(54) **Esterified propoxylated glycerin fat substitute compositions resistant to gastrointestinal side effects**
Esterifierter propoxylierter Glycerin enthaltende Fettsatzzusammenstellung ohne Magen- und Darmennebenscheinungen
Compositions de substituant de graisse comprenant une glycérine estérifiée propoxylée et résistant aux effets gastroentériques secondaires

(30) Priority: 20.05.1992 US 886538
(43) Date of publication of application: 24.11.1993
(73) Proprietor: ARCO Chemical Technology, L.P., Greenville, Delaware 19807 (US)
(72) Inventor: Masten, Lawrence W., West Chester, Pennsylvania 19382 (US)
(74) Representative: Cropp, John Anthony David

(56) References cited:
- EP-A- 0 254 457
- EP-A- 0 353 928
- EP-A- 0 356 255
- EP-A- 0 506 424
- WO-A-91/10368
- US-A- 4 849 242
- US-A- 5 118 448

## Description

### FIELD OF THE INVENTION:

This invention relates to reduced calorie fat substitutes having a reduced tendency to cause oil leakage, diarrhea, or other undesirable or untoward gastrointestinal side effects when consumed as part of a food composition. More specifically, the invention pertains to esterified propoxylated glycerin fat mimetics which have a particular distribution of fatty acids incorporated therein such that the fat mimetics are better tolerated in the human digestive tract.

### BACKGROUND OF THE INVENTION:

A wide variety of substances have been proposed for use as fat substitutes in food compositions. The chemical structures of such substances are selected such that they are more resistant to breakdown by the metabolic processes of the human digestive system which normally occur upon ingestion of conventional triglyceride lipids. Because of their increased resistance to digestion and absorption, the number of calories per gram available from the fat substitutes is considerably reduced as compared to common vegetable oils, animal fats, and other lipids. The use of such substances thus enables the preparation of reduced calorie food compositions useful in the control of body weight.

EP-A-0 353 928 discloses the preparation of esterified propoxylated glycerin by tranesterification and EP-A-0 356 255 discloses the preparation of esterified propoxylated glycerin from free fatty acids for use as non-caloric food substitutes.

EP-A-0 254 457 discloses esterified epoxide-extended polyols and their use as non-digestible, non-caloric fat substitutes for cooking and in food compositions.

U.S. Pat. No. 4,861,613 describes one class of particularly useful fat substitutes wherein a polyol such as glycerin is alkoxylated with an epoxide such as propylene oxide and then esterified with any of a number of fatty acids to form an esterified alkoxylated polyol. These substances have the physical and organoleptic properties of conventional triglyceride lipids, yet are significantly lower in available (absorbed) calories than edible oils owing to their pronounced resistance towards pancreatic lipase enzymatic hydrolysis. The thermal and oxidative stability of the esterified alkoxylated polyols renders them especially suitable for use in the preparation of reduced. calorie food compositions requiring exposure to high temperatures.

Unfortunately, as a consequence of their hydrolytic stability and low digestibility, the esterified alkoxylated polyols described in U.S. Pat. No. 4,861,613 may tend to cause certain undesirable gastrointestinal side effects when consumed at high levels in the diet. That is, since such esterified alkoxylated polyols are not readily broken down into simpler substances upon ingestion, they largely retain their oily, fat-like character and pass through the digestive tract in substantially unaltered form. Non-digestible fat substitutes in general often function as laxatives in much the same manner as mineral oil. Problems with diarrhea, leakage of the fat substitute through the anal sphincter, separation of the fat substitute as an oil from the excreted fecal matter, and shortened bowel transition times resulting in gastrointestinal discomfort can occur as a result of the non-digestibility of the fat substitutes. Other fat substitutes which are similarly resistant towards digestion are known to produce the same gastrointestinal side effects. Examples include sucrose polyester which is esterified with up to 8 fatty acid groups; see U.S. Pat. Nos. 3,954,976, 4,005,195, 4,005,196, and 5,006,360. Obviously, such problems will greatly limit the maximum usage level of these substances which can be tolerated in various food compositions, thereby constraining the amount of conventional triglyceride and the number of calories which can be removed from certain foods.

Various solutions to these problems have been proposed, but none are entirely satisfactory. For example, the structure of the ester linkages in an esterified polyether may be selected such that the linkages are more readily hydrolyzed by enzymes present in the digestive tract, as described in U.S. Pat. No. 4,949,242. The esterified polyether is thereby converted into a polyether, which is considerably less lipophilic and fat-like in character than the original esterified polyether owing to the loss of the fatty acid groups and the generation of free hydroxyl groups, and free fatty acids. The liberated fatty acids are metabolized and digested in the same manner as the fatty acids derived from enzymatic hydrolysis of conventional triglycerides, however, The esterified polyether will thus have a greater caloric availability than might be desired in a fat substitute. Similar results would be expected where certain of the fatty acid ester groups are attached directly to the polyol (e.g., glycerin) residue rather than to oxyalkylene segments, as where the fat substitute is not highly alkoxylated (see Table I of U.S. Pat. No. 4,861,613) or where special synthetic methods are employed as described in copending European patent application 92302662.9 and U.S. Application Serial No. 600,462, filed October 19 1990.

It is apparent that there exists a need for a fat substitute or fat mimetic which is highly resistant towards hydrolysis and absorption, thus providing significant caloric reduction as compared to a conventional triglyceride edible lipid, and yet which also does not cause the same undesired gastrointestinal side effects as prior art fat substitutes when consumed at high dietary levels.

### SUMMARY OF THE INVENTION:

This invention provides a fatty acid-esterified propoxylated glycerin composition useful as a reduced caloric fat substitute resistant to gastrointestinal side effects having an average number of oxypropylene units per equivalent of glycerin of from 3 to 20, a fatty acid acyl group content such that at least 40 mole percent of the fatty acid acyl groups in the composition are derived from a C₂₀-C₂₄ saturated linear fatty acid, and a solid fat index at 27°C as measured by dilatometry of at least 30.

Example 35 of EP-A-0 353 928 describes the transesterification of a propoxylated glycerin containing on average 8 oxypropylene units per molecule with an amount of methyl tetracosanoate in excess of that required to react with each hydroxyl group of the propoxylated glycerin. The product of this process is excluded from this invention.

Additionally provided is a reduced calorie food product having a fat component comprising said fatty acid-esterified propoxylated glycerin composition.

Another aspect of the present invention is a reduced calorie fat component resistant to gastrointestinal side effects and useful for the preparation of food products comprising an edible triglyceride and said fatty-acid esterified propoxylated glycerin composition.

The invention in addition furnishes a method of preparing a reduced calorie food composition having a fat component resistant to gastrointestinal side effects, said method comprising formulating said food composition with such a fatty acid-esterified propoxylated glycerin composition.

Also provided by this invention is a fatty acid-esterified propoxylated glycerin composition useful as a reduced calorie fat substitute resistant to gastrointestinal side effects having an average number of oxypropylene units per equivalent of glycerin of from 5 to 16, a fatty acid acyl group content such that at least 60 mole percent of the fatty acid acyl groups in the composition are derived from behenic acid, and a solid fat index at 27°C measured by dilatometry of at least 40.

The present invention also furnishes a fatty acid-esterified propoxylated glycerin composition obtainable by alkoxylating glycerin with from 3 to 20 equivalents of propylene oxide per equivalent of glycerin, preferably under base-catalyzed conditions, to yield a propoxylated glycerin composition and esterifying the propoxylated glycerin composition with at least one fatty acid or fatty acid equivalent, selected such that the resulting fatty acid-esterified propoxylated glycerin composition has a solid fat index at 27°C as measured by dilatometry of at least 30 and a fatty acid acyl group content wherein at least 40 mole percent of the fatty acid acyl groups are derived from a C₂₀-C₂₄ saturated linear fatty acid.
Preferably, at least about 80% of the hydroxyl groups of the propoxylated glycerin composition are esterified.

### DETAILED DESCRIPTION OF THE INVENTION:

In order for the fatty acid-esterified propoxylated glycerin composition to have minimal tendency to cause gastrointestinal side effects, it is essential that the solid fat index at 27°C (80°F) be at least 30 as measured by dilatometry. Preferably, the solid fat index at this temperature is at least 40. The solid fat index is determined in accordance with A.O.C.S. Official Method Cd 10-57. As will be explained in more detail subsequently, the solid fat index at 27°C may be readily controlled as desired by varying the C₂₀-C₂₄ saturated linear fatty acyl group content, the iodine number (which is a measure of the level of unsaturation or carbon-carbon double bonds), the average number of fatty acid acyl group carbons per equivalent of glycerin, and the average number of oxypropylene units per equivalent of glycerin in the fatty acid-esterified propoxylated glycerin composition. To achieve optimum mouthfeel and other organoleptic characteristics, it is highly desirable that the solid fat index be less than 80 (more preferably, less than 70) at 21°C (room temperature) and less than 40 (preferably, less than 30) at 37°C (body temperature). Compositions having a relatively high solid fat index at body temperature may have a pronounced waxy taste or texture and thus may not be acceptable for use in high proportions in certain food compositions. The solid fat index should be less than 80 at room temperature so that the properties of a food composition containing the fat mimetic most closely resemble the properties of a conventional food composition. Due to the methods by which the fat mimetic compositions of this invention may be conveniently prepared, said compositions are typically mixtures of individual esterified propoxylated glycerin compounds.

The esterified propoxylated glycerin compounds comprising the esterified propoxylated glycerin compositions of this invention contain covalently linked glyceryl residues, oxypropylene units, and fatty acid acyl 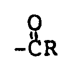 groups. The glyceryl residue may have the generic structure 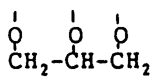 and is derived from glycerin 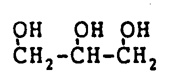 or a glycerin equivalent. The oxypropylene units are generally interspersed between glyceryl residues and the acyl groups and have the structure 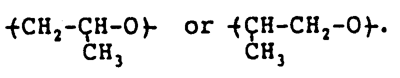 Typically, more than one oxypropylene unit may be present between an oxygen of an individual glyceryl residue and an acyl group such that a polyoxypropylene unit is created. However, a single "branch" or "arm" of an esterified propoxylated glycerin compound may contain only one oxypropylene unit. Some portion of the acyl groups may be attached directly to the glyceryl residue, without an intervening oxypropylene unit, although it is preferred that less than 25% of the acyl groups in the overall composition be attached in this manner. In a preferred embodiment, the average number of oxypropylene units per equivalent of glycerin in the esterified alkoxylated glycerin composition is from 3 to 20. More preferably, an average of from 4 to 16 oxypropylene units per equivalent of glycerin is present. The presence of oxypropylene units is critical, as the oxypropylene units help to lower the melting point of the compositions thereby improving the mouthfeel and melting characteristics as compared to analogous compositions not containing oxypropylene units.

In order to maximize the resistance of the esterified propoxylated glycerin composition towards pancreatic lipase enzyme-catalyzed hydrolysis, it will generally be desirable that the oxypropylene units adjacent to the acyl groups be oriented such that predominantly secondary rather than primary ester linkages are created. That is, the methyl group should be located on the carbon atom attached to the oxygen atom forming part of the ester linkage as follows: 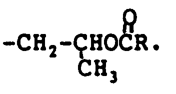 Preferably, at least 80% of the ester linkages in the overall compositions are secondary. Most preferably, at least 95% of the ester linkages in the overall composition are secondary.

In a preferred embodiment of this invention, the esterified propoxylated glycerin composition has a porcine pancreatic lipase hydrolysis rate of below 20% as compared to an olive oil standard. Preferably, the relative hydrolysis rate is below 10%. Methods of measuring porcine pancreatic lipase hydrolysis rate are described in U.S. Pat. No. 4,861,613.

The average number of oxypropylene units in the esterified propoxylated glycerin composition must not be so low as to result in a high proportion of the acyl groups being attached directly to glyceryl residues since such directly attached acyl groups will be nearly as susceptible to enzymatic cleavage as the acyl groups in a conventional fully digestible triglyceride, thus reducing the usefulness of the composition as a low calorie fat substitute. At the same time, the average number of oxypropylene units per equivalent of glycerin should generally not exceed about 20 since the resulting compositions may be substantially higher in viscosity when in liquid form than a conventional triglyceride lipid and thus less useful as direct substitutes for such a lipid owing to the differences in formulation which may be required.

The solid fat index at 27°C may be adjusted as needed by varying the average number of oxypropylene units per equivalent of glycerin (degree of propoxylation) in the composition. At a constant fatty acid acyl group content (i.e., if the relative proportions of the different acyl groups present are fixed), the solid fat index will increase as the degree of propoxylation is decreased and will decrease as the degree of propoxylation is increased. Expressed a different way, decreasing the degree of propoxylation shifts the melting range (the temperature range over which the composition changes from predominantly solid to predominantly liquid) to a higher temperature while increasing the degree of propoxylation shifts the melting range to a lower temperature. As the proportion of C₂₀-C₂₄ saturated linear fatty acid acyl groups decreases (i.e., as the average number of acyl group carbons per equivalent of glycerin decreases) or as the iodine number of the composition increases (as a result of increasing the proportion of unsaturated fatty acid acyl groups present), the average number of oxypropylene units per glycerin will need to be decreased to maintain the solid fat index at 27°C above the critical value of 30. If a particular fatty acid-esterified propoxylated glycerin composition has an undesirably high solid fat index at 21°C or 37°C, it is possible to readily improve the organoleptic qualities of the composition by increasing the degree of propoxylation provided that the solid fat index at 27°C of the resulting composition is still at least 30.

The selection of specific structures and distributions of the acyl groups in the esterified propoxylated glycerin compositions of this invention is critical to the capacity of such substances to function as low calorie fat mimetics having a reduced tendency to promote gastrointestinal side effects upon ingestion. It has been unexpectedly discovered that the incorporation of a certain proportion of acyl groups derived from long chain C₂₀-C₂₄ saturated linear fatty acids dramatically and effectively changes the properties of an esterified propoxylated glycerin composition such that it no longer tends to act as a laxative when consumed in relatively large quantities. At the same time, the esterified propoxylated glycerin composition is highly resistant to digestion and metabolic breakdown and consequently contributes only a low level of calories to the diet. Thus, the use of the esterified propoxylated glycerin composition of this invention avoids having to forsake a desired reduction in caloric content in order to attain a fat substitute which is acceptably tolerated in the digestive tract.

At least 40 mole percent of the fatty acid acyl groups in the esterified propoxylated glycerin composition must be derived from a C₂₀-C₂₄ saturated linear fatty acid. More preferably, at least 60 mole percent of the acyl groups are so derived. "Derived from" in this context means that the acyl group has a long chain hydrocarbyl structure analogous to that present in a C₂₀-C₂₄ saturated linear fatty acid. As will be explained subsequently, the esterified propoxylated glycerin composition may actually be prepared using either a fatty acid or a fatty acid derivative such as a fatty acid ester, fatty acid halide, or fatty acid anhydride. Generally speaking, it will be desirable to increase the proportion of acyl groups derived from C₂₀-C₂₄ saturated linear fatty acids as the average number of oxypropylene segments in the composition is increased in order to most favorably influence the non-laxative properties of the esterified propoxylated glycerin composition.

The incorporation of acyl groups derived from C₂₀-C₂₄ saturated linear fatty acids not only reduces the tendency of the esterified propoxylated glycerin composition to provoke undesirable gastrointestinal side effects, but also appears to generally lower the available calories in the fat substitute as compared to analogous esterified propoxylated glycerin compositions which do not contain long chain fatty acid acyl groups. That is, the esterified propoxylated glycerin compositions of this invention are apparently even more resistant to digestion, absorption, and metabolic breakdown than similar prior art compounds. By careful selection of the fatty acid acyl groups and the degree of propoxylation, it is thus possible to achieve an optimum caloric reduction in the esterified propoxylated glycerin compositions.

The C₂₀-C₂₄ saturated fatty acid is linear (i.e., non-branched) and preferably contains only one carboxylic acid functionality. The acyl group may thus correspond to the general structure 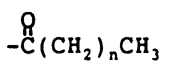 wherein n is an integer of from 18 to 22. The value of n is most conveniently an even number (e.g., 18, 20, or 22) since the corresponding fatty acids are readily available at low cost from natural sources such as edible triglycerides. Specific illustrative fatty acids suitable for use as this component of the esterified propoxylated glycerin compositions include, but are not limited to eicosanoic (arachidic) acid, heneicosanoic acid, docosanic (behenic) acid, tricosanoic acid, and tetracosanoic (lignoceric) acid. Mixtures of these C₂₀-C₂₄ saturated fatty acids may also be utilized to advantage. The long chain saturated fatty acid most preferred for use is behenic acid (i.e., the acyl group has the structure 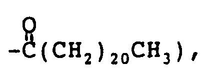 both because it effectively imparts desirable anti-laxative properties to an esterified propoxylated glycerin composition and because it is readily available by hydrogenation of the erucic acid derived from the triglycerides present in rapeseed oil.

While all of the acyl groups in the esterified propoxylated glycerin composition may be derived from a C₂₀-C₂₄ saturated linear fatty acid, up to 60 mole % of the acyl groups may be derived from other C₈-C₂₄ fatty acids. Preferably, the proportion of such other acyl groups is less than 40 mole %. Generally speaking, the incorporation of acyl groups which are relatively short in length (C₈-C₁₈), unsaturated, and/or branched will tend to lower the solid fat index at 27°C of the resulting esterified propoxylated glycerin.

The fatty acids which optionally may be used in combination with the required C₂₀-C₂₄ saturated linear fatty acids may be any of the known fatty acids such as caprylic acid, pelargonic acid, capric acid, lauric acid, palmitic acid, stearic acid, oleic acid, cetoleic acid, myristic acid, palmitoleic acid, gadoleic acid, erucic acid, rincinoleic acid, linoleic acid, linolenic acid, myristoleic acid, eleostearic acid, arachidonic acid, or mixtures or hydrogenated derivatives of these acids. Preferably, linear monocarboxylic acids containing from 0 to 5 double bonds are employed.

The proportions and chemical structures of the fatty acid acyl groups in the fat mimetic compositions of this invention should be selected such that the solid fat index at 27°C as measured by dilatometry does not drop below 30 (more preferably, 40). At a constant degree of propoxylation, increasing the relative proportion of C₂₀-C₂₄ saturated linear fatty acid acyl groups will elevate the solid fat index at 27°C. As described previously, up to 60 mole percent of the fatty acid acyl groups in the composition may be derived from fatty acids other than C₂₀-C₂₄ saturated linear fatty acids. Decreasing the proportion of unsaturated fatty acid acyl groups present will result in an increase in the solid fat index at 27°C. For this reason, the iodine number should be less than 50 centigrams I₂ per gram of the composition and more preferably is less than 25. However, it has been discovered that the incorporation of a minor amount of unsaturated fatty acid acyl groups tends to decrease the solid fat index at 21°C, thus improving the mouthfeel of the composition while maintaining the solid fat index at 27°C above the critical value of 30. The relative proportion of unsaturated fatty acid acyl groups present may be conveniently measured by determination of the iodine number (also referred to as iodine value) of the composition using standard wet chemical methods such as AOCS method Cd 1-25. Increasing the number of different fatty acid acyl groups present in the composition tends to have the same favorable effect on the melting profile of the composition (i.e., lowering the solid fat index at 21°C while at the same time keeping the solid fat index at 27°C above 30). For this reason, it may be advantageous to employ a fatty acid mixture of the type obtainable by splitting or hydrolysis of a natural triglyceride such as soybean fatty acids, coconut fatty acids, corn oil fatty acids, tallow fatty acids, cottonseed oil fatty acids, peanut oil fatty aids, safflower seed oil fatty acids, and the like. Decreasing the proportion of short chain (i.e., C₈-C₁₄) fatty acid acyl groups relative to long chain (i.e., C₁₆-C₂₄) fatty acid acyl groups will also serve to increase the solid fat index at 27°C.

To optimize the hydrophobic and fat-like properties of the compositions of this invention, the propoxylated glycerin should be substantially esterified. That is, essentially all of the hydroxyl groups of the propoxylated glycerin composition are preferably reacted with fatty acid or a fatty acid derivative. For this reason, the composition should contain an average of at least 2.5 (more preferably, at least 2.9) fatty acid acyl groups per equivalent of glycerin. The proportion of unreacted hydroxyl groups may be readily determined by standard wet chemical methods such as hydroxyl number.

To minimize the available caloric content of the esterified propoxylated glycerin fat substitutes of this invention, the chemical composition should be selected such that the number average molecular weight is at least about 750. More preferably, the minimum molecular weight is about 1000. In order for the esterified propoxylated glycerin composition to mimic as closely as possible the physical properties of a natural lipid (particularly viscosity when in liquid or melted form), it is also desirable that the number average molecular weight not exceed about 2300. Preferably, the molecular weight is below about 2000.

The esterified propoxylated glycerin fat substitutes of this invention may be prepared using any suitable method. In general, the procedures described in the prior art for synthesizing other esterified propoxylated glycerin compositions will be appropriate for use provided that the necessary C₂₀-C₂₄ saturated linear fatty acids or fatty acid derivatives are employed in the esterification step. Such procedures are described, for example, in U.S. Pat. Nos. 4,861,613 and 4,983,329 and in European Patent Publication No. 353,928. A reduced calorie fat substitute resistant to gastrointestinal side effects comprising a mixture of esterified propoxylated glycerin compounds in accordance with this invention can be obtained by adaptation or modification of the esterification procedures described in the above-mentioned publications. Thus, a propoxylated glycerin is esterified with fatty acid equivalents selected such that at least about 40% (more preferably, at least about 60%) of the fatty acid acyl groups in the resulting mixture are derived from C₂₀-C₂₄ saturated linear fatty acids. The remaining fatty acid acyl groups, if any, may be derived from C₈-C₂₄ fatty acids other than C₂₀-C₂₄ saturated fatty acids. As is explained in more detail in the above-mentioned publications, either fatty acids or fatty acid equivalents such as fatty acid esters, fatty acid halides, or fatty acid anhydrides may actually be employed in the esterification. The C₂₀-C₂₄ saturated linear fatty acid acyl groups may also be introduced by using C₂₀-C₂₄ unsaturated linear fatty acids in the esterification step and then hydrogenating the esterified propoxylated glycerin composition to increase the proportion of C₂₀-C₂₄ saturated linear fatty acid acyl groups to the desired level.

Particularly preferred embodiments of the fat substitute compositions of this invention are as follows.

A composition having an average of from 7 to 9 oxypropylene units per equivalent of glycerin and an iodine number less than 10, wherein 40 to 95 percent of the fatty acid acyl groups are derived from behenic acid and 5 to 60 percent of the fatty acid acyl groups are derived from C₁₂-C₂₄ saturated or unsaturated fatty acids other than behenic acid.

A composition having an average of from 4 to 6 oxypropylene units per equivalent of glycerin and an iodine number less than 15, wherein 50 to 70 percent of the fatty acid acyl groups are derived from arachidic acid, behenic acid, lignoceric acid or mixtures thereof and 30 to 50 percent of the fatty acid acyl groups are derived from C₁₂-C₂₄ saturated or unsaturated fatty acids other than arachidic acid, behenic acid, and lignoceric acid.

A composition having an average of from 4 to 12 oxypropylene units per equivalent of glycerin and an iodine number less than 10, wherein the fatty acid acyl groups are derived from a mixture of hydrogenated high erucic rapeseed oil fatty acids containing at least 40 mole percent behenic acid.

A composition having an average of from 6 to 10 oxypropylene units per equivalent of glycerin and an iodine number of from 5 to 25, wherein 60 to 90 percent of the fatty acid acyl groups are derived from arachidic acid, behenic acid, lignoceric acid or mixtures thereof and 10 to 40 percent of the fatty acid acyl groups are derived from a mixture of soybean oil, corn oil, peanut oil, cottonseed oil, safflower seed oil or sunflower seed oil fatty acids.

Fatty acid esterified propoxylated glycerin compositions in accordance with the invention may also be prepared by separately synthesizing compositions having different fatty acid acyl group contents and then blending such compositions, provided that the resulting blend composition has an average number of oxypropylene units per equivalent of glycerin of from 3 to 20, a fatty acid acyl group content such that at least 40 mole percent of the fatty acid acyl groups are derived from a C₂₀-C₂₄ saturated linear fatty acid, and a solid fat index at 27° as measured by dilatometry of at least 30.

The esterified propoxylated glycerin compositions of this invention may be used as partial or total (100 %) replacements for conventional lipids in any edible fat-containing food composition. The amount of the fat mimetic employed is sufficient to effectively reduce the available calories of the food composition as compared to a food composition prepared using an equivalent amount (weight or volume) of a conventional fully digestible triglyceride lipid alone. Preferably, at least about 10 percent (more preferably, at least about 25 percent by weight) of the total fat-like component of the food composition is comprised of the esterified propoxylated glycerin composition.

The triglyceride lipid admixed with the esterified propoxylated glycerin composition may be any of the known edible fatty acid triglycerides available from natural or synthetic sources. These edible fatty acid triglycerides include, but are not limited to, fats and oils such as tallow, soybean oil, cottonseed oil, coconut oil, palm kernel oil, corn oil, fish oil, lard, butterfat, olive oil, palm oil, peanut oil, safflower seed oil, cocoa butter, sesame seed oil, rapeseed oil, sunflower seed oil, as well as fully or partially hydrogenated derivatives and mixtures of these triglycerides. While the esterified propoxylated glycerin composition may be combined in any proportion with the triglyceride lipid, weight ratios of from 5:95 to 95:5 are particularly advantageous. The triglyceride lipid may be selected so as to impart a desirable level of calories, flavor, aroma, mouth feel, thermal stability, viscosity, or other such property to the blend and to the resulting food composition.

Certain of the esterified propoxylated glycerin compositions of this invention may tend to have a waxy or gritty mouthfeel as a consequence of their high solids content. To eliminate or minimize any such unpleasant organoleptic properties, the compositions are preferably combined with one or more liquid triglyceride lipids. The lipid may be any of the fatty acid triglycerides discussed hereinabove provided it has a complete melting point of 37°C (body temperature) or below (more preferably, a complete melting point of 25°C or below). The esterified propoxylated glycerin composition is advantageously dispersed in the form of fine particles in a matrix of the liquid triglyceride lipid. Preferably, the particles have an average size of 25 microns or less (more preferably, 10 microns or less). The weight ratio of liquid triglyceride lipid to esterified propoxylated glycerin is most desirably from about 0.5:1 to about 10:1 (more preferably from about 1.5:1 to about 4:1). To obtain dispersions of this type, the esterified propoxylated glycerin composition and liquid triglyceride lipid may be combined in slurry form and the resulting slurry subjected to milling. The temperature during the milling operation, which reduced the particle size of the esterified propoxylated glycerin composition to the desired level, should be maintained below (preferably, at least 15°F below) the complete melting point of the esterified propoxylated glycerin composition (the minimum temperature at which the composition has a solid-fat index of 0).

The fat substitute composition of this invention can replace, in full or in part, a triglyceride lipid in a cooking oil, frying oil, salad oil, or shortening, for example. Additional uses include combining the esterified propoxylated glycerin composition with other foodstuff ingredients to form food compositions such as frozen desserts (e.g., sherbet, ice cream, frozen yogurt, milk shakes), baked goods (cakes, doughnuts, muffins, brownies, breads, pies, rolls, pastries, cookies, biscuits, crackers), nut butters (peanut butter), dairy products (margarine, sour cream, coffee lighteners, cheese, cheese spreads, flavored dips, filled cream, filled milk), mayonnaise, salad dressing, savory snacks (potato chips, corn chips, cheese puffs, pretzels), fried foods (fried poultry, fritters, fried pies, fried vegetables such as french fried potatoes, fried fish), reformed and comminuted meats (lunch meats, sausage, hot dogs, hamburger), pet foods, meat and egg substitutes or extenders, whipped toppings, gravies and other sauces, frostings, fillings, icings, cocoa butter replacements or blends, candies (especially those normally containing fatty ingredients such as chocolate or peanut butter), soups, and dry baking mixes (for muffins, cakes, pancakes, waffles, brownies, and the like). Owing to the fat-like properties and stability of the esterified propoxylated glycerin compositions, minimum reformulation of standard food compositions will generally be required. The viscosity, melting profile, yield point, hardness, thixotropic area, liquid/solid stability, solid fat index, and other physical properties of the esterified propoxylated glycerin composition are preferably selected such that they mimic as closely as possible the analogous properties of the conventional triglyceride being replaced.

Illustrative ingredients which may be used in combination with the fat mimetics of this invention include carbohydrates (flour, starches, sugars, celluloses), edible lipids (triglycerides), proteins (from animal or vegetable sources), vitamins, antioxidants, emulsifiers, thickeners, preservatives, colorants, flavors, fragrances, sugar substitutes (saccharin, aspartame, sucralose, cyclamates, and the like), other fat substitutes or fat mimetics (for example, sucrose polyester or caprenin), water, milk, spices, eggs, and the like. Oil-in-water or water-in-oil emulsions can be readily prepared by combining water, the esterified propoxylated glycerin composition, and other ingredients such as emulsifiers. The esterified propoxylated glycerin compositions of this invention are particularly suitable for the preparation of food compositions requiring exposure to elevated temperatures. Unlike other proposed fat substitutes such as proteinacious macrocolloids or certain polysaccharide-based substances requiring water to render them fat-like in texture, the fat mimetics of this invention are exceptionally stable thermally and do not readily decompose or lose their fat-like properties when heated. The fat mimetics thus may readily be utilized in deep fat frying applications to prepare fried foods such as savory snacks, fried chicken, fried fish, french fries, and the like since they will function as effective heat transfer media (that is, they will transmit heat rapidly and uniformly to the food being fried and also provide crisping).

From the foregoing description, one skilled in the art can readily ascertain the essential characteristics of this invention and, without departing from the spirit and scope thereof, can make various changes and modifications of the invention to adapt it to various usages, conditions, and embodiments.

The following examples further illustrate the fat mimetics and food compositions of this invention, but are not limitative of the invention in any manner whatsoever.

### EXAMPLES

### PREPARATION AND EVALUATION OF ESTERIFIED PROPOXYLATED GLYCERIN COMPOSITIONS

For comparative purposes, the following series of esterified propoxylated glycerin compositions differing in acyl group content was prepared by esterifying a propoxylated glycerin containing an average of approximately 8 equivalents of propylene oxide (i.e., about 8 oxypropylene segments) per glyceryl residue with different fatty acids or mixtures of fatty acids.
- *EPG - 1:: prepared using a mixture of soybean fatty acids having the approximate composition 0.1% myristic acid, 9.8% palmitic acid, 2.5% stearic acid, 0.4% palmitoleic acid, 28.9% oleic acid, 50.7% linoleic acid, and 6.5% linolenic acid (solid fat index at 27°C = 0; iodine number = 100 g I₂/100g).
- *EPG - 2:: prepared using 95% stearic acid (solid fat index at 27°C = 3; iodine number <1g I₂/100g).
- EPG - 3:: prepared using a mixture of approximately 85% behenic acid and 15% stearic acid (solid fat index at 27°C = 76; iodine number <1g I₂/100g).
- EPG - 4:: prepared using 3 parts of a mixture of approximately 85% behenic acid and 15% stearic acid and 1 part of a mixture of soybean fatty acids having the composition indicated in the EPG - 1 description above (solid fat index at 21°C = 40; iodine number = 12.5g I₂/100g).
- EPG - 5:: prepared using a mixture of approximately 64% behenic acid and 36% stearic acid (solid fat index at 27°C = 71; iodine number <1g I₂/100g).

*comparative example

Rat feeding studies were performed wherein one of the esterified propoxylated glycerin compositions described hereinabove was introduced at varying levels in the diet of the rats. Ten male rats per group were employed. The rats were allowed free access to these diets for a period of two weeks, with the feces of the rats being scored daily for oil leakage, soft feces, and diarrhea.

Feeding EPG - 1 and EPG - 2 (esterified propoxylated glycerin compositions not containing any appreciable amount of acyl groups derived from C₂₀-C₂₄ saturated linear fatty acids) at 5% dietary levels did not result in any significant gastrointestinal side effects (Tables I and II). At higher dietary levels of these fat substitutes, however, the observed frequency of oil anal leakage and soft fecal pellets increased to unacceptable levels. When 15% or more of these esterified propoxylated glycerins was present in the diet, for example, nearly all of the animals exhibited anal leakage of the fat substitute. These comparative studies demonstrate the problems which can be encountered when high concentrations of prior art esterified propoxylated glycerin compositions are introduced into the diet.

When esterified propoxylated glycerin compositions in accordance with this invention were employed (Table III and IV), significantly fewer difficulties with gastrointestinal side effects were observed when the fat substitute was present at high levels in the diet. EPG - 3, which contained a high proportion (85%) of acyl groups derived from a C₂₀-C₂₄ saturated linear fatty acid, was the most effective in controlling oil leakage and diarrhea. EPG - 5, which contained acyl groups derived from stearic acid in addition to acyl groups derived from behenic acid, performed somewhat better than EPG - 4, wherein the acyl groups other than those derived from behenic acid were derived from soybean fatty acids. This demonstrates the advantage of selecting the acyl groups other than those derived from C₂₀-C₂₄ saturated linear fatty acids to also be saturated rather than unsaturated. However, even EPG - 4 exhibited significantly less of a tendency than either EPG - 1 or EPG - 2 to provoke diarrhea (as measured by the softness of the fecal pellets). The performance of EPG - 4 could be readily improved by decreasing the average number of oxypropylene units per glycerin (for example, 6 rather than 8).

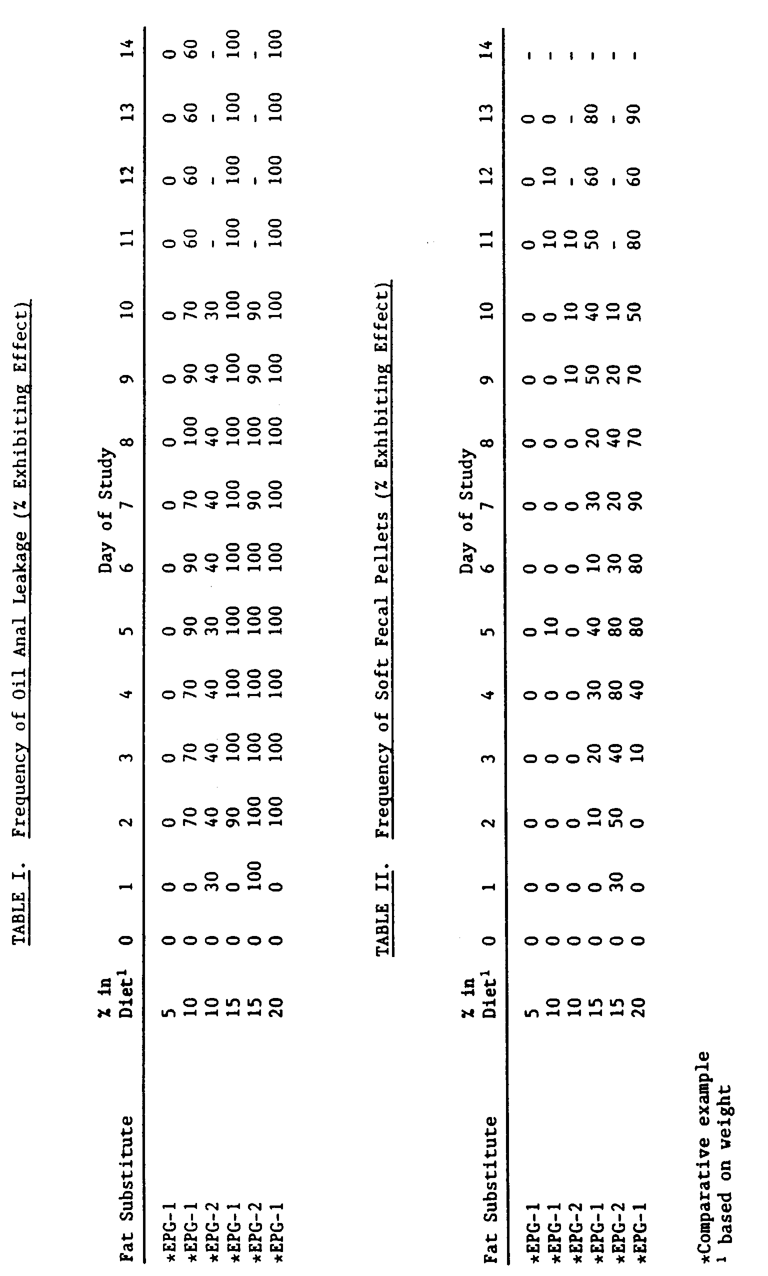

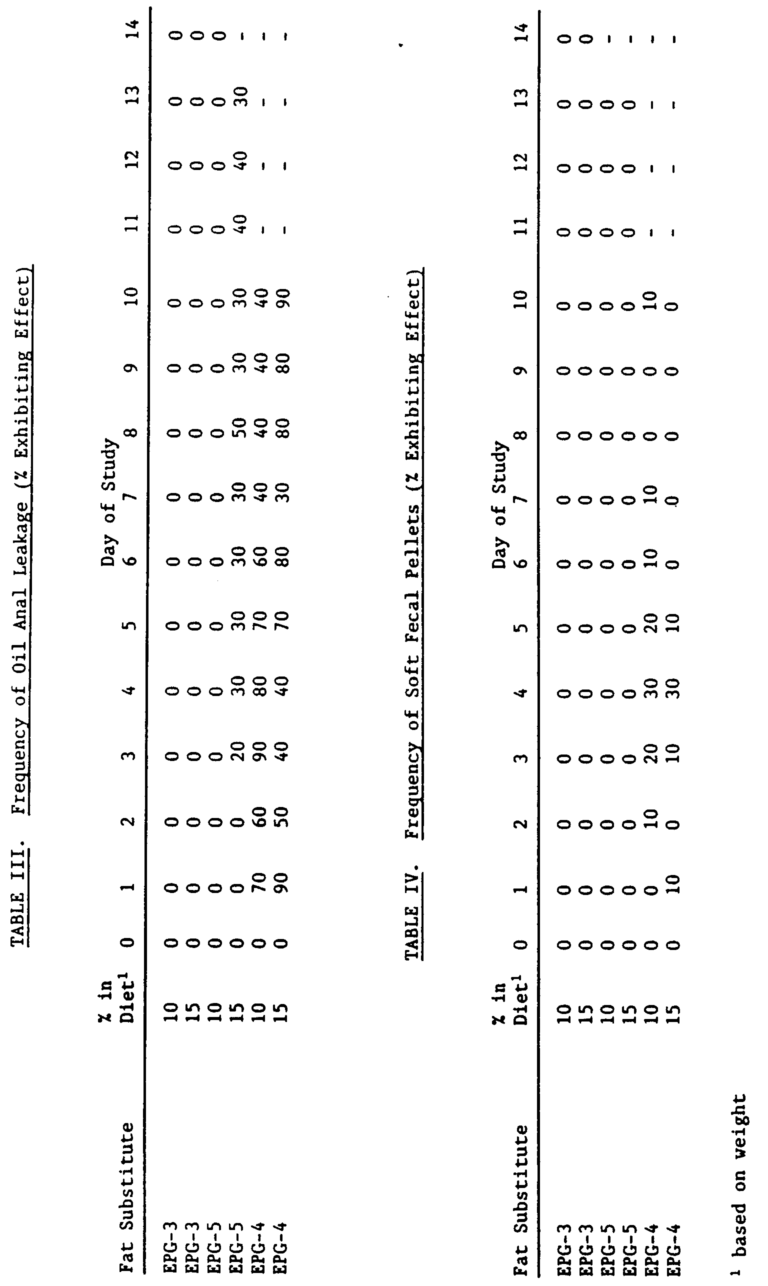

Adapting the propoxylation and esterification procedures described in U.S. Pat. No. 4,983,329, the following illustrative esterified propoxylated glycerins having compositions in accordance with the present invention are prepared. These substances are expected to be similar to conventional edible triglyceride lipids in physical and organoleptic properties, substantially resistant to digestion and hence lower in available calories than triglyceride lipids, and consumable at relatively high concentrations in the diet owing to their reduced tendency to cause gastrointestinal side effects.
- EPG - 7:: an esterified propoxylated glycerin composition containing an average of 8 oxypropylene units per glyceryl residue and prepared using a 42.5:57.5 molar ratio of behenic acid and stearic acid (solid fat index at 27°C = 61; iodine number <1g I₂/100g).
- EPG - 8:: an esterified propoxylated glycerin containing an average of 8 oxypropylene units per glyceryl residue and prepared using 3 parts of a mixture of approximately 85% behenic acid and 15% stearic acid and 1 part of a mixture of coconut fatty acids (C₈-C₁₈ fatty acids, predominantly lauric acid). Solid fat index at 27°C = 37; iodine number <1g I₂/100g.
- EPG - 9:: an esterified propoxylated glycerin composition containing an average of 12 oxypropylene units per glyceryl residue and prepared using a mixture of fully hydrogenated rapeseed oil fatty acids (approximately 55 wt. % behenic acid, with the balance being predominantly stearic acid).
- EPG - 10:: an esterified propoxylated glycerin containing an average of 5 oxypropylene units per glyceryl residue and prepared using a hydrogenated mixture of 9 parts rapeseed oil fatty acids and 1 part soybean fatty acids (solid fat index at 27°C ca. 65). Fatty acid acyl group composition: C14:0 0.1 wt.%; C16:0 3.4 wt.%; C18:0 31.6 wt.%; C18:1 3.4 wt.%; C18:2 0.4 wt.%; C20:0 8.6 wt.%; C22:0 50.4 Wt.%; C24:0 1.6 wt.%; unknown 0.5 wt.%.
- EPG - 11:: an esterified propoxylated glycerin containing an average of 6 oxypropylene units per glyceryl residue and prepared using a 1:1:1 molar ratio of behenic acid, eicosanoic acid, and lauric acid.
- EPG - 12:: an esterified propoxylated glycerin containing an average of 15 oxypropylene units per glyceryl residue and prepared a 3:1 molar ratio of lignoceric acid and palmitic acid.

Additional fatty acid-esterified propoxylated glycerin compositions in accordance with the invention were prepared by separately synthesizing fatty acid-esterified propoxylated glycerin compositions having different fatty acid acyl group contents and then blending said compositions in various proportions. For comparative purposes, other blends having compositions falling outside the scope of the invention were similarly prepared.
- EPG - 13:: a 1:1 blend of EPG-3 and EPG-2 (solid fat index at 27°C = 40; iodine number <1g I₂/100g).
- *EPG - 14:: a 1:3 blend of EPG-3 and EPG-2 (solid fat index at 27°C = 25; iodine number <1g I₂/100g).
- *EPG - 15:: a 1:7 blend of EPG-3 and EPG-2 (solid fat index at 27°C = 10; iodine number <1g I₂/100g).
- EPG - 16:: a 1:1 blend of EPG-3 and EPG-1 (solid fat index at 27°C = 35 iodine number = 50g I₂/100g).
- *EPG - 17:: a 1:3 blend of EPG-3 and EPG-1 (solid fat index at 27°C = 16; iodine number = 75g I₂/100g).
- *EPG - 18:: a 1:7 blend of EPG-3 and EPG-1 (solid fat index at 27°C = 7; iodine number = 87.5g I₂/100g).
- EPG - 19:: a 1:1 blend of EPG-3 and an esterified propoxylated glycerin prepared by esterifying a propoxylated glycerin containing an average of approximately 8 equivalents of propylene oxide per glyceryl residue with a mixture of coconut fatty acids (solid fat index at 27°C = 54; iodine number <1g I₂/100g).

*comparative example

Rat feeding studies were performed using these compositions and the same procedure described hereinabove. The results of these studies are presented in Tables V-VIII. The use of fatty acid-esterified propoxylated glycerin compositions containing a relatively high proportion of C₂₀-C₂₄ saturated linear fatty acid acyl groups and having a solid fat index at 27°C of more than 30 was found to lead to significantly less frequent problems with gastrointestinal side effects.

To confirm the results observed in the rat feeding studies, five male purebred beagles (6.4-12.3 kg) were administered either corn oil (as a control) or fatty acid-esterified propoxylated glycerin compositions in combination with Certified Canine Diet #5007 (product of Purina Mills, Inc.). Portions of approximately 275 to 300 g of each diet offered in this study were made available for about 4 hours per day. For the first seven days of the study, all of the dogs received a diet containing 11.25% corn oil (2.76 g/kg/day). No leakage or phase separation of lipid from feces defecated during that time period was observed.

Over a six week period, the five dogs were given a diet containing EPG-1 (solid fat index at 27°C = 0) at a 5.63% level (1.54 g/kg/day) for two weeks, then a diet containing EPG-1 at an 8.44% level (2.27 g/kg/day) for two weeks, and finally a diet containing EPG-1 at an 11.25% level (3.12 g/kg/day) for two weeks. No leakage or phase separation of oil from the excreted fecal matter was observed at the lowest EPG-1 dietary level. However, all of the animals exhibited perianal soiling and fecal oil separation at the highest EPG-1 dietary level. The frequency of perianal soiling and oil in the collection pans was somewhat less at the intermediate EPG-1 dietary level, although the frequency of fecal oil separation was equivalent to that observed at the 11.25% level.

To demonstrate the benefits of using the fatty acid-esterified propoxylated glycerin compositions of this invention as fat substitutes, the dogs were then fed a diet containing 11.25% EPG-4 (solid fat index at 27°C = 40; 3.12 g/kg/day) for two weeks and then a diet containing 16.89% EPG-4 (4.89 g/kg/day) for an additional two weeks. The first EPG-4 diet was introduced immediately after the conclusion of the test period described hereinabove wherein an 11.25% EPG-1 diet had been administered. After a clear-out period of a few days, no evidence of oil leakage from the anal sphincter or phase separation of the EPG-4 from the feces was observed.

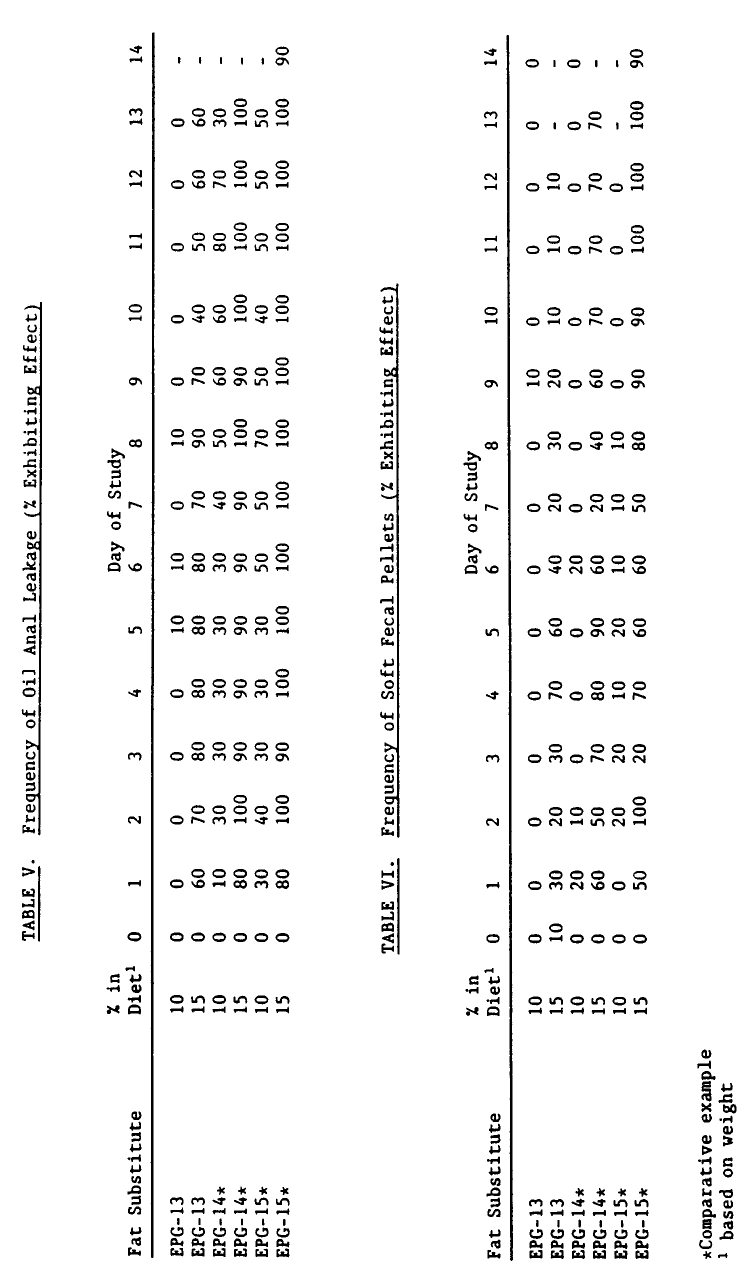

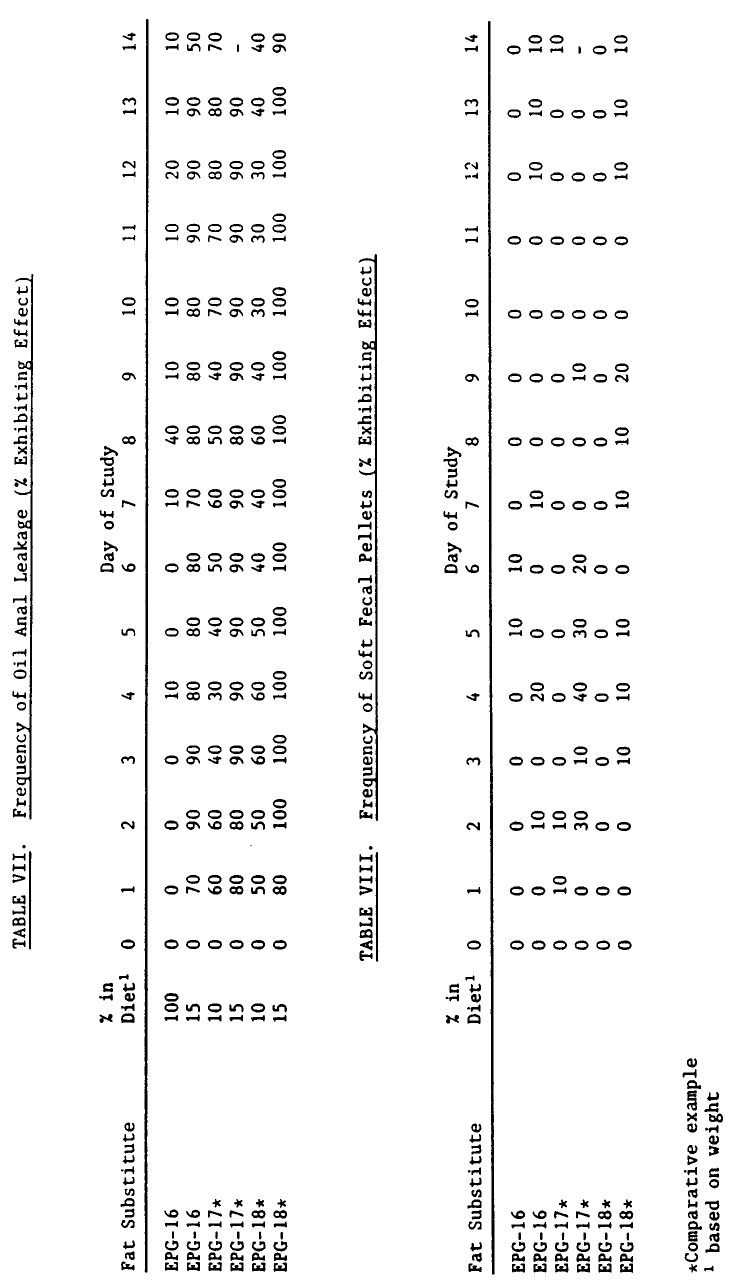

### ILLUSTRATIVE FOOD PRODUCTS

### French Fries

This example demonstrates the preparation of reduced calorie french fries using the esterified propoxylated glycerin composition of this invention.

Potatoes are pared and then cut lengthwise in strips approximately 3/8 inch in width. EPG - 3 is heated to 360°F in a suitable deep-fat cooking vessel; sufficient esterified propoxylated glycerin composition is employed to provide a layer at least about 2 inches deep in the vessel. The cut potato strips are then placed in the hot block copolymer for 6 to 7 minutes or until crisp and golden. Drain on paper towels and sprinkle with salt.

The french fries thus prepared are expected to be similar in taste, odor, and appearance to french fries prepared using a conventional triglyceride oil. However, their available caloric content is significantly reduced owing to the digestion- and absorption-resistant character of the esterified propoxylated glycerin composition.

### Margarine

This example demonstrates the preparation of a reduced calorie margarine using the esterified propoxylated glycerin composition of this invention in combination with conventional digestible triglyceride lipids. The margarine has the following formulation:

| Ingredient | Weight % |
|---|---|
| fat phase | 82.7 |
| monoglyceride¹ | 0.2 |
| salt | 1.0 |
| flavoring, colorants | 0.2 |
| water | balance to 100% |

| | |
|---|---|
| ¹ "Admul 6203" (product of Unimills) | |

The composition of the fat phase is as follows:

| Ingredient | Weight % |
|---|---|
| soybean oil | 13.0 |
| palm oil | 10.0 |
| partially hydrogenated palm oil (slip melting point 44°C) | 17.0 |
| EPG - 5 | 60.0 |

The margarine is prepared by admixing the separately prepared aqueous phase and fat phase in a pre-emulsion vessel and subsequently passing the pre-emulsion, having a temperature of about 55°C, through two scraped surface heat exchangers (units 1 and 2) and a resting tube (unit 3). The processing conditions used are as follows:

| Unit | 1 | 2 | 3 |
|---|---|---|---|
| rotation speed, rpm | 1000 | 800 | - |
| jacket temperature, °C | -8 | 22 | - |
| exit temperature, °C | 20 | 26 | 25 |

The solidified product is extruded into standard one-quarter pound sticks and refrigerated. The margarine thus prepared is expected to be similar in taste, odor, mouth feel, texture, and physical properties to a conventional margarine prepared using only fully digestible triglyceride lipids, yet will contain significantly fewer available calories owing to the esterified propoxylated glycerin composition present. The margarine may be consumed at higher dietary level without causing serious gastrointestinal side effects as compared to a margarine prepared using an esterified propoxylated glycerin composition lacking the minimum content of acyl groups derived from C₂₀-C₂₄ saturated linear fatty acids required by this invention.

### Frosting

A reduced calorie frosting suitable for use on baked goods is prepared using the following ingredients:

| Ingredient | Amount |
|---|---|
| EPG - 4 | 15 tablespoons |
| butter | 5 tablespoons |
| confectioner's sugar | 10 cups |
| skim milk | 20 tablespoons |
| vanilla | 10 teaspoons |

Cream EPG - 4, butter, and sugar well, then add the skim milk and vanilla and mix until the mixture is smooth and spreads easily.

### Frozen Dairy Dessert

An esterified propoxylated glycerin composition of this invention having a melting point between about 32°C and 35°C (18 parts by weight) is homogenized with skim milk (82 parts by weight) in a conventional dairy homogenizer to yield a "filled cream" composition. The "filled cream" composition (68 parts by weight) is combined with condensed skim milk (15 parts), sugar (15 parts) or an amount of aspartame or other artificial sweetener to provide an equivalent degree of sweetness, gelatin (0.5 parts), flavor (1.0 parts), and food-grade colorant (0.25 parts) to produce a frozen dairy dessert mix which is processed in the normal manner to yield a reduced calorie frozen dairy dessert.

### Raisin Cookies

Reduced calorie raisin cookies are prepared by creaming sugar (24.3 parts by weight) and invert sugar (20.0 parts) with EPG - 5 (13.0 parts), whole eggs (2.0 parts), and vanilla (0.1 parts). A mixture of flour (13.7 parts), sodium bicarbonate (0.1 parts), monocalcium phosphate (0.1 parts), and salt (0.1 parts), is then added in portions to the creamed mixture and mixed well. Raisins (19.0) parts) are then added to the cookie dough mixture. The mixture is formed into balls containing about 1 tablespoon of dough per ball. The dough balls are placed on a greased cookie sheet and baked for 10-15 minutes in a preheated 375°C oven to yield reduced calorie raisin cookies.

### Peanut Butter

A reduced calorie peanut butter is prepared by mixing EPG - 4 (35.0 parts by weight) with peanut flavor (2.0 parts by weight) and then adding corn syrup solids (12.0 parts), salt (1.0 parts), and high fructose corn syrup (10.0 parts) to the resulting mixture with good agitation. When these ingredients are well blended, defatted peanut flour (40.0 parts) is added and mixed thoroughly to yield a reduced calorie peanut spread.

### Pound Cake

Reduced calorie pound cakes are prepared using the following ingredients:

| Ingredient | Quantity |
|---|---|
| cake flour | 1 lb. |
| reduced calorie margarine (prepared as described hereinabove) | 1 lb. |
| table sugar | 1 lb. |
| whole eggs | 9 |
| vanilla | 2 tablespoons |

Cream reduced calorie margarine containing EPG - 5 well, then add sugar gradually and continue beating until light and fluffy. Add eggs two at a time, beating well after each addition. Add flavoring. Add flour gradually and beat until smooth. Pour mixture into three greased bread loaf pans and bake about 1 1/2 hours in a preheated 300°C oven.

### Doughnuts

Reduced calorie doughnuts are prepared using the following ingredients:

| Ingredients | Quantity |
|---|---|
| flour | 2 cups |
| sugar | 1/2 cup |
| salt | 1 teaspoon |
| baking powder | 3 teaspoons |
| cinnamon | 1/4 teaspoon |
| grated nutmeg | dash |
| melted reduced calorie margarine (described hereinabove) | 2 tablespoons |
| skim milk | 1/2 cup |
| egg, beaten | 1 |

Sift dry ingredients, then add melted reduced calorie margarine. Add the milk to the egg and combine the mixtures. After kneading lightly, roll lightly to a thickness of about 1/4 inch. Cut with doughnut cutter.

Heat 3-4 inches of a 4:1 mixture of EPG - 3 and shortening (partially hydrogenated vegetable oil) to a temperature of about 360°F. Cook doughnuts until evenly browned, turning often. Remove from frying mixture and drain on absorbent paper.

### Fried Fish

A fritter batter is prepared using the following ingredients:

| Ingredient | Quantity |
|---|---|
| flour | 1 1/3 cups |
| salt | 1 teaspoon |
| pepper | 1/4 teaspoon |
| reduced calorie margarine (prepared as described hereinabove) | 1 tablespoon |
| egg yolks, beaten | 2 |
| flat beer | 3/4 cup |

All the ingredient except the beer are combined and mixed well. The beer is then added gradually, stirring constantly. The batter is allowed to rest covered and refrigerated 3-12 hours before use.

Small fish or pieces of fish not thicker than 1 inch are dipped in the fritter batter and then fried for 5-8 minutes until golden brown in a 3-4 inch deep 4:1 mixture of EPG - 5 and corn oil which has been preheated to 370°F. Drain on absorbent paper.

### Fried Chicken

Reduced calorie fried chicken is prepared by cutting a fresh broiler-fryer into pieces, seasoning with salt and pepper, then coating with cracker crumbs. After standing 10 minutes, dip in slightly beaten egg and again in crumbs. Fry in a 3-4 inch deep 2:1 mixture of EPG - 3 and soybean oil at a temperature of about 350°F for about 10-15 minutes or until golden brown.

## Claims

1. A fatty acid-esterified propoxylated glycerin composition useful as a reduced calorie fat substitute resistant to gastrointestinal side effects characterised in that said composition has an average number of oxypropylene units per equivalent of glycerin of from 3 to 20, a fatty acid acyl group content such that at least 40 mole percent of the fatty acid acyl groups in the composition are derived from a C₂₀-C₂₄ saturated linear fatty acid, and a solid fat index at 27°C as measured by dilatometry of at least 30, but excluding the product obtainable by transesterifying propoxylated glycerin containing on average 8 oxypropylene units per molecule with an amount of methyl tetracosanoate which is in excess of that required to react with each of the hydroxy groups of the propoxylated glycerin.

2. A fatty acid-esterified propoxylated glycerin composition useful as a reduced calorie fat substitute resistant to gastrointestinal side effects characterised in that said composition has an average number of oxypropylene units per equivalent of glycerin of from 3 to 20, a fatty acid acyl group content such that at least 40 mole percent of the fatty acid acyl groups in the composition are derived from a C₂₀-C₂₄ saturated linear fatty acid, and values for solid fat indexes as measured by dilatometry as follows:
at 21°C less than 80
at 27°C at least 30
at 37°C less than 40.

3. A composition as claimed in claim 1 or claim 2 characterised in that the C₂₀-C₂₄ saturated linear fatty acid is selected from arachidic acid, behenic acid, lignoceric acid, and mixtures thereof.

4. A composition as claimed in claim 1, claim 2 or claim 3 characterised in that at least 60 mole percent of the fatty acid acyl groups are derived from the C₂₀-C₂₄ saturated linear fatty acid.

5. A composition as claimed in any one of the preceding claims characterised in that the solid fat index at 27°C is at least 40.

6. A composition as claimed in any one of the preceding claims characterised in that it has an iodine number less than 50.

7. A composition as claimed in any one of the preceding claims characterised in that up to 60 mole percent of the fatty acid acyl groups in the composition are derived from C₈-C₂₄ fatty acids selected from the group consisting of caprylic acid, capric acid, lauric acid, palmitic acid, stearic acid, oleic acid, cetoleic acid, myristic acid, palmitoleic acid, gadoleic acid, erucic acid, rincinoleic acid, linoleic acid, linolenic acid, myristoleic acid, eleostearic acid, arachidonic acid, and mixtures thereof.

8. A composition as claimed in any one of the preceding claims characterised in that it has a porcine pancreatic lipase hydrolysis rate of below 20% compared to olive oil.

9. A composition as claimed in any one of the preceding claims characterised in that it has an average of at least 2.5 fatty acid acyl groups per equivalent of glycerin.

10. A composition as claimed in any one of the preceding claims characterised in that at least 85 percent of the fatty acid acyl groups are bonded to oxypropylene units through a secondary ester linkage.

11. A composition as claimed in any one of the preceding claims characterised in that it has an average number of oxypropylene units per equivalent of glycerin of from 4 to 16, a fatty acid acyl group content such that at least 60 mole percent of the fatty acid acyl groups in the composition are derived from behenic acid, and a solid fat index at 27°C as measured by dilatometry of at least 40.

12. A composition as claimed in claim 11 characterised in that up to 40 mole percent of the fatty acid acyl groups in the composition are derived from C₈-C₂₄ fatty acids selected from the group consisting of caprylic acid, capric acid, lauric acid, palmitic acid, stearic acid, oleic acid, cetoleic acid, myristic acid, palmitoleic acid, gadoleic acid, erucic acid, rincinoleic acid, linoleic acid, linolenic acid, myristoleic acid, eleostearic acid, arachidonic acid, and mixtures thereof.

13. A reduced calorie food product having a fat component, said fat component comprising a fatty acid-esterified propoxylated glycerin composition as claimed in any one of claims 1 to 12.

14. The food product of claim 13 which additionally contains a non-fat ingredient.

15. A reduced calorie fat component resistant to gastrointestinal side effects and useful for the preparation of food products, said fat component comprising an edible triglyceride and a fatty-acid esterified propoxylated glycerin composition as claimed in any one of claims 1 to 12.

16. A reduced calorie fat component as claimed in claim 15 characterised in that the edible triglyceride is selected from tallow, soybean oil, cottonseed oil, coconut oil, palm kernel oil, corn oil, fish oil, lard, butterfat, olive oil, palm oil, peanut oil, safflower seed oil, cocoa butter, sesame seed oil, rapeseed oil, sunflower seed oil, and fully or partially hydrogenated derivatives thereof, and mixtures thereof.

17. A reduced calorie fat component as claimed in claim 15 or claim 16 characterised in that the fatty acid-esterified propoxylated glycerin composition comprises at least 25 percent by weight of the fat component.

18. A method of preparing a reduced calorie food composition having a fat component resistant to gastrointestinal side effects, said method comprising formulating said food composition with a fatty acid-esterified propoxylated glycerin composition as claimed in any one of claims 1 to 12.

## Patentansprüche

1. Mit einer Fettsäure veresterte, propoxylierte Glycerin-Zusammensetzung, die als Fettersatz mit weniger Kalorien nützlich ist und keine gastrointestinalen Nebenwirkungen aufweist, dadurch gekennzeichnet, daß die Zusammensetzung eine durchschnittliche Anzahl an Oxypropyleneinheiten pro Glycerinäquivalent von 3 bis 20, einen solchen Gehalt an Fettsäureacylgruppen, daß mindestens 40 Mol-% der Fettsäureacylgruppen in der Zusammensetzung von einer gesättigten, linearen C₂₀-C₂₄-Fettsäure abgeleitet sind und einen Festfettindex bei 27°C, gemessen durch Dilatometrie, von mindestens 30 aufweist, wobei das Produkt ausgeschlossen ist, das durch Umestern von propoxyliertem Glycerin, das durchschnittlich 8 Oxypropyl-Einheiten pro Molekül enthält, mit einer Methyltetracosanoat-Menge erhältlich ist, die gegenüber der Menge, die zur Umsetzung mit jeder Hydroxy-Gruppe des propoxylierten Glycerins erforderlich ist, im Überschuß vorhanden ist.

2. Mit einer Fettsäure veresterte, propoxylierte Glycerin-Zusammensetzung, die als Fettersatz mit weniger Kalorien nützlich ist und keine gastrointestinalen Nebenwirkungen aufweist, dadurch gekennzeichnet, daß die Zusammensetzung eine durchschnittliche Anzahl an Oxypropyleneinheiten pro Glycerinäquivalent von 3 bis 20, einem solchen Gehalt an Fettsäureacylgruppen, daß mindestens 40 Mol-% der Fettsäureacylgruppen der Zusammensetzung von einer in gesättigten, linearen C₂₀-C₂₄-Fettsäure abgeleitet sind und Werte für Festfettindizes, gemessen durch Dilatometie, wie folgt aufweist:
bei 21°C weniger als 80
bei 27°C mindestens 30
bei 37°C weniger als 40.

3. Zusammensetzung nach Anspruch 1 oder 2, dadurch gekennzeichnet, daß die gesättigte, lineare C₂₀-C₂₄-Fettsäure ausgewählt ist unter Arachidonsäure, Behensäure, Lignocerinsäure und Gemischen davon.

4. Zusammensetzung nach Anspruch 1, Anspruch 2 oder Anspruch 3, dadurch gekennzeichnet, daß mindestens 60 Mol-% der Fettsäureacylgruppen von der gesättigten, linearen C₂₀-C₂₄-Fettsäure abgeleitet sind.

5. Zusammensetzung nach einem der vorhergehenden Ansprüche, dadurch gekennzeichnet, daß der Festfett-Index bei 27°C mindestens 40 beträgt.

6. Zusammensetzung nach einem der vorhergehenden Ansprüche, dadurch gekennzeichnet, daß sie eine Jodzahl von Weniger als 50 aufweist.

7. Zusammensetzung nach einem der vorhergehenden Ansprüche, dadurch gekennzeichnet, daß bis zu 60 Mol-% der Fettsäureacylgruppen in der Zusammensetzung von C₈-C₂₄-Fettsäuren abgeleitet sind, die ausgewählt sind aus der Gruppe bestehend aus Caprylsäure, Caprinsäure, Laurinsäure, Palmitinsäure, Stearinsäure, Ölsäure, Cetoleinsäure, Myristinsäure, Palmitoleinsäure, Gadoleinsäure, Erucasäure, Rizinusölsäure, Linolsäure, Linolensäure, Myristoleinsäure, Elaeostearinsäure, Arachidonsäure und Gemischen davon.

8. Zusammensetzung nach einem der vorhergehenden Ansprüche, dadurch gekennzeichnet, daß sie eine Schweinepankreaslipase-Hydrolyserate von unter 20%, verglichen mit Olivenöl, aufweist.

9. Zusammensetzung nach einem der vorhergehenden Ansprüche, dadurch gekennzeichnet, daß sie durchschnittlich mindestens 2,5 Fettsäureacylgruppen pro Glycerinäquivalent aufweist.

10. Zusammensetzung nach einem der vorhergehenden Ansprüche, dadurch gekennzeichnet, daß mindestens 85% der Fettsäureacylgruppen durch eine sekundäre Esterbindung an die Oxypropyleneinheiten gebunden sind.

11. Zusammensetzung nach einem der vorhergehenden Ansprüche, dadurch gekennzeichnet, daß sie eine durchschnittliche Anzahl an Oxypropyleneinheiten pro Glycerinäquivalent von 4 bis 16, einem solchen Gehalt an Fettsäureacylgruppen, daß mindestens 60 Mol-% der Fettsäureacylgruppen in der Zusammensetzung von Behensäure abgeleitet sind und einen Festfett-Index bei 27°C, gemessen durch Dilatometrie, von mindestens 40 aufweist.

12. Zusammensetzung nach Anspruch 11, dadurch gekennzeichnet, daß bis zu 40 Mol-% der Fettsäureacylgruppen in der Zusammensetzung von C₈-C₂₄-Fettsäuren abgeleitet sind, die ausgewählt sind aus der Gruppe bestehend aus Caprylsäure, Caprinsäure, Laurinsäure, Palmitinsäure, Stearinsäure, Ölsäure, Cetoleinsäure, Myristinsäure, Palmitoleinsäure, Gadoleinsäure, Erucasäure, Rizinusölsäure, Linolsäure, Linolensäure, Myristoleinsäure, Elaeostearinsäure, Arachidonsäure und Gemischen davon.

13. Kalorienreduziertes Nahrungsmittelprodukt mit einer Fettkomponente, wobei die Fettkomponente eine mit einer Fettsäure veresterte, propoxylierte Glycerin-Zusammensetzung nach einem der Ansprüche 1 bis 12 enthält.

14. Nahrungsmittelprodukt nach Anspruch 13, das zusätzlich einen Nichtfett-Inhaltsstoff enthält.

15. Kalorienreduziete Fettkomponente, die keine gastrointestinalen Nebenwirkungen aufweist und zur Herstellung von Nahrungsmittelprodukten nützlich ist, wobei die Fettkomponente ein eßbares Triglycerid und eine mit einer Fettsäure veresterte, propoxylierte Glycerin-Zusammensetzung nach einem der Ansprüche 1 bis 12 enthält.

16. Kalorienreduzierte Fettkomponente nach Anspruch 5, dadurch gekennzeichnet, daß das eßbare Triglycerid ausgewählt ist, unter Talg, Sojaöl, Baumwollsamenöl, Kokosnußöl, Palmkernöl, Maisöl, Fischöl, Schweinefett, Butterfett, Olivenöl, Palmöl, Erdnußöl, Saffloröl, Kakaobutter, Sesamsamenöl, Rapsöl, Sonnenblumenöl und vollständig oder teilweise hydrierten Derivaten davon, und Gemischen davon.

17. Kalorienreduzierte Fettkomponente nach Anspruch 15 oder 16, dadurch gekennzeichnet, daß die mit einer Fettsäure veresterte, propoxylierte Glycerin-Zusammensetzung mindestens 25 Gew.-% der Fettkomponente umfaßt.

18. Verfahren zur Herstellung einer kalorienreduzieten Nahrungsmittelzusammensetzung, die eine Fettkomponente enthält, die keine gastrointestinalen Nebenwirkungen aufweist, wobei das Verfahren das Formulieren der Nahrungsmittel-zusammensetzung mit einer propoxylierten Glycerin-Zusammensetzung, die mit einer Fettsäure verestert ist, nach einem der Ansprüche 1 bis 12 umfaßt.

## Revendications

1. Composition de glycérine estérifiée par un acide gras et propoxylée, utile en tant que substituant de graisse, à teneur calorique réduite, résistant aux effets gastroentériques secondaires, caractérisée en ce que ladite composition a un nombre moyen d'unités oxypropylène par équivalent de glycérine compris entre 3 et 20, une teneur en groupes acyle d'acide gras telle qu'au moins 40 moles pour cent des groupes acyle d'acide gras dans la composition, sont dérivés d'un acide gras linéaire saturé c₂₀-c₂₄ , et un indice de graisse solide à 27°C, d'au moins 30, mesuré au dilatomètre, mais excluant le produit pouvant être obtenu par transestérification de glycérine propoxylée contenant en moyenne 8 unités oxypropylène par molécule, avec une quantité de tétracosanoate de méthyle qui est en excès par rapport à celle requise pour réagir avec chacun des groupes hydroxy de la glycérine propoxylée.

2. Composition de glycérine estérifiée par un acide gras et propoxylée, utile en tant que substituant de graisse, à teneur calorique réduite, résistant aux effets gastroentériques secondaires, caractérisée en ce que ladite composition a un nombre moyen d'unités oxypropylène par équivalent de glycérine compris entre 3 et 20, une teneur en groupes acyle d'acide gras telle qu'au moins 40 moles pour cent des groupes acyle d'acide gras dans la composition, sont dérivés d'un acide gras linéaire saturé C₂₀-C₂₄, et dont les valeurs d'indices de graisse solide, mesurées au dilatomètre, sont les suivantes :
- à 21°C, moins de 80
- à 27°C, au moins 30
- à 37°C, moins de 40.

3. Composition selon la revendication 1, ou la revendication 2, caractérisée en ce que l'acide gras linéaire saturé C₂₀-C₂₄ est choisi parmi l'acide arachidique, l'acide béhénique, l'acide lignocérique, et les mélanges de ceux-ci.

4. Composition selon la revendication 1, la revendication 2, ou la revendication 3, caractérisée en ce qu'au moins 60 moles pour cent des groupes acyle d'acide gras sont dérivés d'acide gras linéaire saturé C₂₀-C₂₄.

5. Composition selon l'une quelconque des revendications précédentes, caractérisée en ce que l'indice de graisse solide à 27°C est d'au moins 40.

6. Composition selon l'une quelconque des revendications précédentes, caractérisée en ce qu'elle a un indice d'iode inférieur à 50.

7. Composition selon l'une quelconque des revendications précédentes, caractérisée en ce que jusqu'à 60 moles pour cent des groupes acyle d'acide gras sont dérivés d'acides gras C₈-C₂₄ choisis au sein du groupe comprenant l'acide caprylique, l'acide caprique, l'acide laurique, l'acide palmitique, l'acide stéarique, l'acide oléique, l'acide cétoléique, l'acide myristique, l'acide palmitoléique, l'acide gadoléique, l'acide érucique, l'acide ricinoléique, l'acide linoléique, l'acide linolénique, l'acide myristoléique, l'acide éléostéarique, l'acide arachidonique, et les mélanges de ceux-ci.

8. Composition selon l'une quelconque des revendications précédentes, caractérisée en ce qu'elle a une vitesse d'hydrolyse par la lipase pancréatique porcine de moins de 20% comparé à l'huile d'olive.

9. Composition selon l'une quelconque des revendications précédentes, caractérisée en ce qu'elle a en moyenne 2,5 groupes acyle d'acide gras, par équivalent de glycérine.

10. Composition selon l'une quelconque des revendications précédentes, caractérisée en ce qu'au moins 85 % des groupes acyle d'acide gras sont liés aux unités oxypropylène par une liaison ester secondaire.

11. Composition selon l'une quelconque des revendications précédentes, caractérisée en ce qu'elle a un nombre moyen d'unités oxypropylène par équivalent de glycérine compris entre 4 et 16, une teneur en groupes acyle d'acide gras telle qu'au moins 60 moles pour cent des groupes acyle d'acide gras dans la composition sont dérivés de l'acide béhénique, et un indice de graisse solide à 27°C d'au moins 40, mesuré au dilatomètre.

12. Composition selon la revendication 11, caractérisée en ce que jusqu'à 40 moles pour cent de groupes acyle d'acide gras dans la composition, sont dérivés d'acides gras C₈-C₂₄ choisis au sein du groupe comprenant l'acide caprylique, l'acide caprique, l'acide laurique, l'acide palmitique, l'acide stéarique, l'acide oléique, l'acide cétoléique, l'acide myristique, l'acide palmitoléique, l'acide gadoléique, l'acide érucique, l'acide ricinoléique, l'acide linoléique, l'acide linolénique, l'acide myristoléique, l'acide éléostéarique, l'acide arachidonique, et les mélanges de ceux-ci.

13. Aliment à teneur calorique réduite, ayant un composant gras, ledit composant gras comprenant une composition de glycérine estérifiée par un acide gras et propoxylée, selon l'une quelconque des revendications 1 à 12.

14. Produit alimentaire selon la revendication 13, qui contient en plus un ingrédient non gras.

15. Composant gras à teneur calorique réduite, résistant aux effets gastroentériques secondaires, et utile pour la préparation de produits alimentaires, ledit composant gras comprenant un triglycéride comestible, et une composition de glycérine estérifiée par un acide gras et propoxylée, selon l'une quelconque des revendications 1 à 12.

16. Composant gras à teneur calorique réduite, selon la revendication 15, caractérisé en ce que le triglycéride comestible est choisi parmi le suif, l'huile de soja, l'huile de graine de coton, l'huile de noix de coco, l'huile de cerneau de palme, l'huile de maïs, l'huile de poisson, le lard, le beurre, l'huile d'olive, l'huile de palme, l'huile d'arachide, l'huile de graine de Carthamus, le beurre de cacao, l'huile de graine de sésame, l'huile de graine de colza, l'huile de graine de tournesol, et les dérivés totalement ou partiellement hydrogénés, et leurs mélanges.

17. Composant gras à teneur calorique réduite selon la revendication 15, ou la revendication 16, caractérisée en ce que la composition de glycérine estérifiée par un acide gras et propoxylée, comprend au moins 25 % en poids, de composant gras.

18. Méthode de préparation d'une composition alimentaire à teneur calorique réduite ,ayant un composant résistant aux effets gastroentériques secondaires, ladite méthode comprenant la formulation de ladite composition alimentaire avec une composition de glycérine estérifiée par un acide gras et propoxylée, selon l'une quelconque des revendications 1 à 12.
